# EUROPEAN PATENT APPLICATION

(11) **EP 4 245 754 A1**
(43) Date of publication of application: **20.09.2023**
(21) Application number: 22162328.3
(22) Date of filing: 15.03.2022
(51) Int. Cl.: C07D 251/60, B01J 10/00, C07C 273/12

(54) **PROCESS FOR THE SYNTHESIS OF MELAMINE**

(71) Applicant: CASALE SA, 6900 Lugano (CH)
(72) Inventor: GAMBA, Simone, 24040 Pagazzano (BG) (IT); FILIPPI, Ermanno, 6976 Castagnola (CH)
(74) Representative: M. Zardi & Co S.A.

(57) **Abstract**

A process for the high-pressure non-catalytic synthesis of melamine from urea wherein various heat inputs of the process are provided electrically, the process comprises reacting urea to form melamine in a synthesis reactor provided with electrical heating elements, the process further comprises heating the melamine melt electrically during transport and/or using electrically produced heat to remove water from melamine crystals.

## Description

### Field of application

The invention relates to a process for the high-pressure non-catalytic synthesis of melamine from urea.

### Prior art

The considerable growth in energy demands and limited fossil fuel sources, together with environmental concerns, have forced the chemical industry to implement more sustainable practices.

Melamine is industrially produced almost exclusively from urea either with a low-pressure gas-phase catalytic process or with a high-pressure liquid-phase non-catalytic process. Both of them are known in the art and widely described in the literature. The high-pressure process is nowadays preferred because it does not need a catalyst and has certain advantages over the gas-phase process, e.g. the liquid phase reaction requires smaller equipment and the melamine offgas can be recycled at a higher pressure.

In the high-pressure process, urea is reacted in a melamine synthesis reactor at a high pressure above 70 bar and a high temperature which is greater than 360 °C and typically in the range 360 to 420 °C to obtain pyrolysis of urea. The pyrolysis of urea produces melamine, carbon dioxide and ammonia. The carbon dioxide and ammonia are gaseous and are normally referred to as melamine offgas. The overall reaction is endothermic, thus needs a heat input.

Said heat input for the conversion of urea into melamine is provided conventionally through a heating circuit including tubes fitted in the melamine synthesis reactor, which carry a heating medium, e.g. molten salts. The heating medium is heated outside the reactor in a gas-fired furnace by indirect heat exchange with combustion fumes obtained by the combustion reaction of natural gas with an oxidant e.g. air.

The melamine is extracted from the reactor as a melamine melt, which contains melamine and some impurities such as melam, melem and oxoaminotriazines. The offgas may be withdrawn from the synthesis reactor separately from the melamine melt or together with the melamine melt, as a biphasic stream, and subsequently separated in a quencher. Offgas are normally recycled to a tied-in urea plant, e.g. directly in gaseous form or after condensation.

The melamine melt is further processed to remove the above-mentioned contaminants, obtaining a purified solution (or slurry) which is subject to crystallization to obtain crystals of melamine of a high purity. The crystals are subjected to finishing process steps like e.g. centrifugation and separation to obtain the final melamine product.

The melamine melt must be continuously heated during transport, to maintain the melt above 370 °C and avoid undesired precipitation of crystals. To this purpose, the melamine melt piping is usually heated with a suitable medium, e.g. a thermal oil. Also, the crystallization process requires a heat input to remove water from the crystals.

For example, in a common embodiment the drying step of melamine crystals is carried out by contacting melamine crystals obtained from the crystallization step with pre-heated air in a so-called flash dryer.

Hence a conventional melamine synthesis process requires several heat inputs including: the heaters fitted in the melamine reactor, the heating system of melamine melt transport pipes; the heating system used in the crystallization process, e.g. the production of hot air used in the flash drier. All the above heat inputs, in the conventional technique, come from the combustion of a fossil fuel, typically from combustion of natural gas.

As a matter of fact, the amount of natural gas used in the melamine process is considerable and, considering the melamine reactor and the melamine drying step, it is estimated that about 80% of natural gas is used to supply energy to the melamine reactor, while the remaining 20% is used during drying operations.

The combustion of a fossil fuel such as natural gas constitutes a major source of carbon dioxide emission into the atmosphere and strongly penalizes the environmental sustainability of the process.

Therefore, in light of the consideration stated above it is highly desirable to provide a more sustainable high-pressure non-catalytic synthesis process for the synthesis of melamine.

### Summary of the invention

The invention aims to solve the drawbacks of the prior art, in particular the invention aims to reduce the environmental impact of the melamine synthesis process.

The aim is reached with a process and plant for the high-pressure non-catalytic synthesis of melamine from urea according to the claims.

The process includes the reaction of urea to form melamine in a reactor comprising a pressure vessel, a first reaction zone and a second reaction zone contained in said pressure vessel, wherein said second reaction zone is coaxially arranged around said first reaction zone and said second reaction zone includes electrical heating elements arranged to provide heat for the endothermic conversion of urea into melamine.

The melamine melt after suitable purification treatments is processed to obtain melamine crystals which are then dried preferably by contacting said crystals with pre-heated air. According to the invention, electrical heating may be used also to maintain the melamine melt at a suitable temperature during transport and/or to remove water from the melamine crystals, preferably for both.

In addition, and according to preferred embodiments, other process steps requiring a heat input, particularly at a temperature of 250 °C or higher, can be heated electrically instead of using a thermal fluid such as thermal oil.

Another aspect of the invention relates to providing, in the second reaction zone, at least two subsets of electric heating elements which can be powered separately and can be controlled independently on the basis of one or more operational parameters of the reactor.

The invention has several advantages including the possibility to reduce or to eliminate the amount of CO₂ emitted into the atmosphere. The melamine reactor, the melamine melt transport lines and the step of drying the melamine crystals, as well as other process steps requiring a heat input, can be heated electrically without requiring the combustion of fossil fuels. Advantageously, when the electric energy is produced from renewable energy sources, e.g. solar energy, no emission of CO₂ into the environment occurs.

Another advantage concerns the simplification of the melamine plant. In particular, the heating circuit that carries the thermal fluid to the melamine reactor can be eliminated. The Capex of the plant is reduced due to the elimination of expensive items like e.g. a molten salt pump. The newly provided electrical heating elements require little or no maintenance so that the cost for maintenance is also reduced.

Another advantage is a faster and more accurate control of the heat input provided to the process. Electrical heating means react faster and can be controlled more precisely compared to a thermal fluid circuit. For example, a pre-heated air used for drying the melamine crystals can be obtained by passing air over one or more resistors which allow a fast and accurate control of the temperature.

### Description of the invention

The invention relates to a process for the high-pressure non-catalytic synthesis of melamine from urea, wherein urea is reacted to form melamine in a melamine synthesis reactor to obtain a melamine melt.

The reaction of urea to yield melamine is performed in a melamine synthesis reactor comprising a first reaction zone and a second reaction zone contained in a pressure vessel, wherein said second reaction zone is coaxially arranged around said first reaction zone. The first reaction zone may be delimited by a duct arranged centrally in the reactor. In a preferred embodiment the liquid flow in said first reaction zone is predominantly directed upward, so that said duct is also termed central riser. The second reaction zone is a heated zone and includes electrical heating elements to provide heat for the endothermic conversion of urea. Preferably said electrical heating elements include resistors.

In certain embodiments, the melamine synthesis reactor may comprise an additional reaction zone wherein gaseous ammonia is introduced and stripping of the offgas from the melamine melt is performed. This additional reaction zone operates as a post-reactor or secondary reactor integrated in the same pressure vessel. Accordingly, the reactor may be regarded as a combined reactor. In other embodiments said additional reaction zone or stripping zone is provided in a separate vessel.

Examples of melamine synthesis reactors which can be used in embodiments of the present invention are described in WO 02/34730 and WO 2015/124409. Preferred solutions concerning the processing of a reactor effluent melamine melt are described in EP 1 444 214 and EP 3 208 264.

Preferably, in the first reaction zone the flow is predominantly directed upward and in the second reaction zone the flow is predominantly directed downward to establish a circulation inside the reactor. Preferably, the offgas are extracted from top of the reactor as a separate stream.

In the high-pressure non-catalytic process, the synthesis of melamine is typically performed at a pressure of at least 70 bar, preferably in the range 100 to 140 bar.

The offgas can be withdrawn directly from the melamine synthesis reactor or from a gas/liquid separator. The reactor offgas may be scrubbed with molten urea or water (aqueous washing) to recover melamine contained therein. Preferably, the offgas are treated in a high-pressure urea scrubber, which operates substantially at the same pressure as the melamine synthesis reactor, wherein the offgas are contacted with the urea feed. The urea melt from the urea scrubber is then sent to the melamine synthesis reactor. Alternatively, the offgas may be scrubbed with an aqueous solution taken from the low-pressure downstream section.

The melamine synthesis reactor and, if provided, the offgas scrubber and/or the post reactor, form a high-pressure synthesis section.

The melamine melt from the high-pressure synthesis section is processed at a lower pressure in a low-pressure section downstream. The processing of the melamine melt typically includes: quenching the melamine melt obtaining a quenched solution; hydrolysis of the solution to remove contaminants (e.g., melam); crystallization of the so obtained purified solution to obtain melamine crystals. A portion of the crystallization mother liquor is then treated in a waste water treatment (WWT) section.

The melamine crystals are then heated to remove water, for example in a suitable heat exchanger or by contacting said melamine crystals with hot air in a flash dryer.

In certain embodiments, the effluent of the reactor is a biphasic stream containing melamine melt and offgas, said stream is quenched and offgas are extracted from the quencher.

The melamine melt is transported under an externally heated condition to maintain the temperature of the melamine melt above 370 °C to avoid precipitation of solid melamine during the transportation from one step to the other. For example, the melamine melt may be transported from the main synthesis reactor to the post-reactor, or from the synthesis section to the downstream quencher.

The heated condition of the melamine melt may be maintained by circulation a thermal fluid that is heated electrically and/or at least some of the heat for removal of water from the melamine crystals may also be produced electrically. For example, said thermal fluid circulates in suitable heating jackets of the melamine melt piping.

In a preferred embodiment, the melamine crystals are dried by a direct contact with hot air and said hot air is heated electrically. In other embodiments, a heat exchanger may be used for heating the crystals and remove water therefrom. For example, a conventional process of drying the melamine crystals may use a steam drier, i.e. a heat exchanger with heat exchange bodies, such as tubes, fed with hot steam. According to an embodiment of the invention said steam drier may be replaced with a heat exchanger using a hot medium, such as thermal oil, which is heated electrically.

Some embodiments of the invention include the production of thermal oil at a temperature of at least 250 °C for one or more thermal users of the melamine synthesis process, said thermal oil being heated electrically. Said thermal oil may be used in some embodiments to heat vessels and/or piping where needed. In certain embodiments, electrically heated thermal oil may be circulated in a jacket of the reactor vessel to provide heat to the reactor. Particularly, an interesting embodiment includes that electrically heated thermal oil is used to preheat mother liquor for a step of hydrolysis in a waste water treatment (WWT) section.

According to a particularly preferred embodiment, the heating of the melamine melt during transport is fully based on electrical heating, i.e. the full amount of heat used to maintain the melt above 370 °C during its transport in pipes is produced electrically. Similarly, in some embodiments, the removal of water from the melamine crystals is fully heated with electrically produced heat.

Hot air for drying the melamine crystals can be produced, preferably, by contacting air with one or more electrical resistors.

According to a particularly preferred embodiment, the heating elements arranged in said second reaction zone are grouped in at least two subsets of heating elements, which can be powered separately. In a preferred embodiment the heating elements are arranged into concentric ranks and each single rank or groups of said ranks may form a subset of heating elements independently operable.

The heating elements are preferably elongate elements so that they act substantially like tubes. In a common embodiment the melamine synthesis reactor is a vertical reactor and the heating elements are vertically elongate.

The process may comprise the step of selectively powering the heating elements, by engaging or disengaging one or more of said heating elements with or from an electrical power source.

The term "engaging" denotes here that an electrical heating element or a subset of the electrical heating elements is powered by said electrical power source. The term "disengaging" indicates that power is interrupted. Hence the reactor may include active heating elements and inactive heating elements.

According to some embodiments, said heating elements are selectively powered on the basis of one or more of: a temperature detected in the second reaction zone of the reactor or a variation thereof, the amount of fresh urea fed to the reactor or a variation thereof, an estimated heat transfer coefficient of one or more of the heating elements or a variation thereof.

According to an embodiment, the reactor may include a number of heating elements that is greater than the number of heating elements required to maintain a desired heat input under design conditions, when the reactor is operated at full load (full capacity). The term full load indicates that the amount of melamine produced in the reactor is comprised between 100 and 110% of the design value.

A redundant number of heating elements in the reactor can be particularly advantageous to compensate for fouling. In particular, fouling results in the deposit of a thick layer of melamine or its derivatives on the electrical heating elements e.g. resistors of the melamine reactor. Said thick layer is responsible of reduced heat transfer coefficient across the heating elements thus potentially leading to a drop in the operative temperature of the reactor. The applicant has found that hot surfaces are more exposed to fouling, therefore the active heating elements are particularly affected.

According to the above, in a particularly interesting application, the process comprises the step of selective powering of some of the heating elements. Accordingly, the heating elements comprise active elements and inactive elements wherein the active elements suffer from the fouling whereas the inactive elements are significantly less affected.

The selection of active heating elements may take into account various parameters and conditions in the melamine synthesis reactor, such as the temperature in the reactor or the feed of urea. The relatively clean inactive elements may be powered after a certain time of operation wherein a loss of performance due to the fouling is detected or estimated.

For example, one or more inactive heating elements may be powered when a temperature reduction in the second reaction zone of the reactor is detected and/or when an increase in urea feed into the reactor is measured and/or when a reduction in the heat transfer coefficient of said one or more subsets of heating elements is estimated.

The engagement (activation) of one or more of said heating elements with the power source during the steady-state operation of the reactor allows compensating for temperature perturbations that occur inside the chemical convert resulting from fouling or load variations.

Related advantages of the above described control include: the reactor can be kept in operation for a longer time and the number of shut down required for maintenance is as well reduced. Therefore, the process of the present invention is not only environmentally friendly but also more efficient.

Generally, the process of the present invention is adapted to implement advanced steps for controlling and regulating the temperature in the melamine reactor thanks to the faster control made possible by the electric heating.

The electrical power source can be configured to provide a constant electrical power across all the subsets of electrical heating elements or it can be configured to modulate the electrical power differently across each subset of heating elements.

Preferably, at least part of the electric energy used for electric heating, in the various embodiments of the invention, is produced from a renewable source. More preferably, all the electric energy used for electric heating is produced from a renewable source.

The invention further relates to a plant for the synthesis of melamine from urea. The plant includes a high-pressure section and a low-pressure section for processing the melamine melt. The high-pressure section includes the melamine synthesis reactor and may include further items such as a post-reactor or a reactor offgas scrubber. The low-pressure section includes a section to remove contaminants (e.g. hydrolysis) and a crystallization section where melamine crystals are obtained from a purified solution and dried to remove water.

The high-pressure synthesis section includes a melamine synthesis reactor provided with electrical heating elements. Said electrical heating elements are preferably electrical resistors. Preferably, the reactor comprises a first reaction zone and a second reaction zone, wherein said second reaction zone is coaxially arranged around said first reaction zone, and said electrical heating elements are provided in the second reaction zone.

The plant may further comprise a circuit for a thermal fluid, such as a thermal oil, which is heated electrically. Said thermal fluid may be used to heat the melamine transport lines and/or to heat pressure vessels or heat exchangers providing a heat input to the overall process. The crystallization section may include a flash drier with electrical resistors to produce hot air, or a heat exchanger fed with an electrically heated medium.

Still in accordance with the invention, a plant for the synthesis of melamine with the high-pressure non-catalytic process comprises at least one melamine synthesis reactor wherein a melamine melt is produced from urea and a low-pressure section wherein melamine melt is processed to obtain melamine crystals, the plant comprising a thermal oil circuit configured to provide heat to one or more thermal users of the plant, said circuit comprising an electric heater wherein said thermal oil is heated electrically. Said thermal users may include one or more of: heating means of a melamine synthesis reactor; heating means of a melamine melt piping; a dryer of the melamine crystals; heating means arranged to heat a crystallization mother liquor for a step of hydrolysis. Said step of hydrolysis of the mother liquor may be performer in a waste water treatment section to remove at least part of melamine and/or OATs contained in said liquor, to obtain a solution that can be discharged.

A further aspect of the invention is a plant for the synthesis of melamine from urea, comprising:
a high-pressure synthesis section wherein melamine is synthesized at a high pressure and a melamine melt is obtained;
a low-pressure section wherein melamine melt is processed and crystals of solid melamine are obtained,
wherein the high-pressure synthesis section includes a synthesis reactor having a reaction zone provided with electrical heating elements;
and also comprising a control system configured to power subsets of the electric elements selectively and on the basis of one or more of: a temperature detected in the second reaction zone of the reactor or a variation thereof, the amount of fresh urea fed to the reactor or a variation thereof, an estimated heat transfer coefficient of one or more of the heating elements or a variation thereof.

Particularly preferably, said reactor comprises two reaction zones coaxially arranged, namely a first reaction zone and a second reaction zone, wherein said second reaction zone is coaxially arranged around said first reaction zone, and said electrical heating elements, which can be separately powered and controlled, are provided in the second reaction zone.

## Claims

1. A process for high-pressure non-catalytic synthesis of melamine from urea, wherein urea is reacted to form melamine in a melamine synthesis reactor, obtaining a melamine melt, said melamine melt is processed to remove impurities and/or offgas to obtain a purified melamine melt, the purified melamine melt is processed to obtain melamine crystals, the melamine crystals are heated to remove water contained therein, wherein the melamine melt obtained in the reactor is transported through subsequent processing steps under an externally heated condition to maintain a temperature above 370 °C to avoid precipitation of solid melamine,
**characterized in that**:
the reaction of urea to form melamine is performed in a reactor comprising a pressure vessel, a first reaction zone and a second reaction zone contained in said pressure vessel, wherein said second reaction zone is coaxially arranged around said first reaction zone;
and **in that** the second reaction zone includes electrical heating elements to provide heat for the endothermic conversion of urea.

2. A process according to claim 1 also **characterized in that** at least one of the following conditions applies:
said heated condition of the melamine melt is maintained by circulation of a thermal fluid, said thermal fluid being heated electrically;
the heating of the melamine crystals to remove water includes electrical heating.

3. A process according to claim 2, wherein the full amount of heat transferred to said thermal fluid is produced electrically and/or the full amount of heat used to remove water from the melamine crystals is produced electrically.

4. A process according to claim 2 or 3 wherein heating the melamine crystals to remove water includes contacting the melamine crystals with hot air, and said hot air is heated electrically.

5. A process according to any of the previous claims, further including the production of thermal oil at a temperature of at least 250 °C for one or more thermal users of the process, said thermal oil being heated electrically.

6. A process according to claim 5, wherein electrically heated thermal oil is used to preheat crystallization mother liquor for a step of hydrolysis.

7. A process according to any of the previous claims, wherein the electric heating elements arranged in said second reaction zone include at least two subsets of electric heating elements which can be powered separately, and the process comprises the step of powering selectively one or more of said subsets of heating elements with an electrical power source.

8. A process according to claim 7 wherein the electric elements are selectively powered on the basis of one or more of: a temperature detected in the second reaction zone of the reactor or a variation thereof, the amount of fresh urea fed to the reactor or a variation thereof, an estimated heat transfer coefficient of one or more of the heating elements or a variation thereof.

9. A process according to claim 7 or 8, wherein said electrical power source is configured to provide a constant electrical power to all said subsets of heating elements or said electrical power source is configured to modulate the electrical power differently across each subset of heating elements.

10. A process according to any of the previous claims, including heating electrically the melamine synthesis reactor during startup.

11. A process according to any of the previous claims wherein at least part of the electric energy for the electric heating is produced from a renewable source.

12. A process according to any of the previous claims wherein offgas are removed from the melamine synthesis reactor separately from a melamine melt, and offgas are scrubbed with molten urea to remove melamine contained therein.

13. A plant for the synthesis of melamine from urea, comprising:
a high-pressure synthesis section wherein melamine is synthesized at a high pressure and a melamine melt is obtained;
a low-pressure section wherein melamine melt is processed and crystals of solid melamine are obtained,
wherein the high-pressure synthesis section includes a synthesis reactor having a reaction zone provided with electrical heating elements;
and also wherein:
the plant comprises a heated piping system adapted to transport the melamine melt while maintaining the melt at a temperature above 370 °C and said heated piping system is heated by a thermal fluid which is heated electrically,
and/or
the low-pressure section includes electrically heated means to remove water from the melamine crystals.

14. A plant according to claim 13 wherein the low-pressure section includes a melamine crystals flash drier which operates with electrically heated air.

15. A plant according to claim 13 or 14 wherein:
the melamine synthesis reactor includes electric heating elements that can be selectively powered, and
the plant comprises a control system configured to provide a selective powering of said heating elements of the reactor on the basis of one or more of: a temperature detected in the second reaction zone of the reactor or a variation thereof, the amount of fresh urea fed to the reactor or a variation thereof, an estimated heat transfer coefficient of one or more of the heating elements or a variation thereof.

16. A plant for the synthesis of melamine with the high-pressure non-catalytic process, comprising at least one melamine synthesis reactor wherein a melamine melt is produced from urea and a low-pressure section wherein melamine melt is processed to obtain melamine crystals, the plant comprising a thermal oil circuit configured to provide heat to one or more thermal users of the plant, said circuit comprising an electric heater wherein said thermal oil is heated electrically, wherein said thermal users include one or more of: heating means of a melamine synthesis reactor; heating means of a melamine melt piping; a dryer of the melamine crystals; heating means arranged to heat a crystallization mother liquor for a step of hydrolysis to at least partly remove melamine and/or OATs contained in said liquor.

17. A plant for the synthesis of melamine from urea, comprising a high-pressure synthesis section wherein melamine is synthesized at a high pressure and a melamine melt is obtained; a low-pressure section wherein melamine melt is processed and crystals of solid melamine are obtained, wherein the high-pressure synthesis section includes a synthesis reactor having a reaction zone provided with electrical heating elements;
and also comprising a control system of the reactor configured to power subsets of the electric elements selectively and on the basis of one or more of: a temperature detected in the second reaction zone of the reactor or a variation thereof, the amount of fresh urea fed to the reactor or a variation thereof, an estimated heat transfer coefficient of one or more of the heating elements or a variation thereof.
